(19) Europäisches Patentamt
European Patent Office
Office européen des brevets

(11) **EP 1 185 315 B1**

(12) **EUROPÄISCHE PATENTSCHRIFT**

(45) Veröffentlichungstag und Bekanntmachung des Hinweises auf die Patenterteilung:
**11.12.2002 Patentblatt 2002/50**

(21) Anmeldenummer: **00935177.6**

(22) Anmeldetag: **07.06.2000**

(51) Int Cl.$^7$: **A61L 27/52**

(86) Internationale Anmeldenummer:
**PCT/EP00/05208**

(87) Internationale Veröffentlichungsnummer:
**WO 00/076561 (21.12.2000 Gazette 2000/51)**

(54) **PLASTISCH VERFORMBARES IMPLANTAT**

PLASTICALLY DEFORMABLE IMPLANT

IMPLANT PLASTIQUEMENT DEFORMABLE

(84) Benannte Vertragsstaaten:
**AT BE CH CY DE DK ES FI FR GB GR IE IT LI LU MC NL PT SE**

(30) Priorität: **12.06.1999 DE 19926889**

(43) Veröffentlichungstag der Anmeldung:
**13.03.2002 Patentblatt 2002/11**

(73) Patentinhaber: **BAUSCH & LOMB INCORPORATED**
**Rochester, New York 14604-2701 (US)**

(72) Erfinder:
• **MENZ, Dirk-Henning**
 **D-86420 Diedorf (DE)**
• **DRESP, Joachim**
 **D-81825 München (DE)**

(74) Vertreter: **Maiwald, Walter, Dr. Dipl.-Chem. et al**
**Maiwald Patentanwalts GmbH**
**Postfach 33 05 23**
**80065 München (DE)**

(56) Entgegenhaltungen:
**WO-A-97/12852      US-A- 4 490 351**
**US-A- 5 573 757**

EP 1 185 315 B1

**Beschreibung**

Die Erfindung betrifft ein plastisch verformbares Implantat nach dem Oberbegriff des Anspruches 1 sowie Verwendungen dieses Implantats zur Herstellung verschiedener ophtalmologischer Hilfsmittel und Arzneimittel.

Derartige plastisch verformbare Implantate finden beispielsweise Anwendung in der Ophthalmologie, insbesondere als Glaskörper- oder Linsenersatz, sowie in der Zahnmedizin beispielsweise zur Füllung von Extraktionshöhlen im Kieferknochen.

Im Bereich der plastischen Chirurgie sind ferner verformbare Implantate bekannt, die jedoch immer aus einer kissenförmigen Umhüllung und einem Implantatwerkstoff zur Füllung bestehen, um eine Abgrenzung zu den umliegenden Geweben bzw. die Bioverträglichkeit zu erreichen.

Für ophthalmologische Anwendungen bekannt sind fluorhaltige Verbindungen in Form von leichtbeweglichen Flüssigkeiten und Präparationen. Hierbei werden insbesondere die für fluorhaltige Verbindungen typischen Eigenschaften, wie hohe Dichte und geringe Oberflächenspannung ausgenutzt. Die bisher zur Anwendung kommenden teilfluorierten bzw. perfluorierten Verbindungen liegen jedoch als einphasige Flüssigkeiten vor. Dadurch bedingt können unterschiedliche Stoffeigenschaften nur insoweit ausgenutzt werden, wie dies durch die Struktur bzw. die inhärenten Eigenschaften der jeweils verwendeten chemischen Verbindungen vorgegeben ist. Deshalb ist es mit den bekannten fluorhaltigen ophthalmologischen Behandlungsmitteln nicht möglich, die oftmals sehr verschiedenen und teilweise gegenläufigen Erfordernisse des Behandlungsmittels mit einem einzigen Stoffbestandteil zu verwirklichen.

So ist beispielsweise bei und nach vitreo retinalen Eingriffen ein Behandlungsmittel erforderlich, welches hervorragende Tamponadeeigenschaften besitzt und gleichzeitig die Möglichkeit des Austausches wasserlöslicher Stoffe bietet, was mit den bekannten ophthalmologischen Behandlungsmitteln aufgrund ihrer Nichtmischbarkeit mit Wasser nicht gleichzeitig realisiert werden kann. Weiterhin wurde versucht, die bei der ophthalmologischen Anwendung von Perfluorcarbonen beobachteten und auf mechanische Wirkungen zurückgeführten Schädigungen an der Retina zu vermeiden, indem Stoffe mit geringerer Dichte eingesetzt wurden, wie sie beispielsweise in der EP 563 446 B1, DE 197 19 280 und der DE 195 36 504 A1 beschrieben werden. Allerdings wurde dabei gleichzeitig die Lipophilie dieser Verbindungen stark erhöht, was zu Penetrationsvorgängen führte. Daraus ergaben sich histologische Veränderungen und auch von Perfluorcarbonen bekannte Nebenwirkungen.

Aus dem Stand der Technik sind weiterhin fluorhaltige Gele aus der Gruppe der Fluorcarbon-Wasser-Emulsionen bekannt. Derartige Emulsionen in Gelform und ihre Verwendungsmöglichkeiten in Medizin und Technik sind beispielsweise in US 5,573,757, in EP 0 340 079 sowie WO 97/03644 beschrieben. Diese Gele bilden Polyaphronstrukturen aus, mit einer kontinuierlichen Minoritätsphase und einer diskontinuierlichen Majoritätsphase. Die Minoritätsphase kapselt dabei die Majoritätsphase vollständig ab und bestimmt daher die wesentlichen Eigenschaften der gesamten Präparation. Gemäß dem Stand der Technik ist zur Herstellung von Präparaten dieses Strukturtyps ein ganz bestimmter Arbeitsablauf einzuhalten. Zudem ist aus dem Stand der Technik bekannt, daß bei derartigen Gelen eine Zerstörung oder Auflösung beispielsweise durch Erhitzen oder mechanischen Druck, irreversibel verläuft, d. h. ein einmal zerstörtes Gel kann seine ursprüngliche Gelstruktur nicht zurückerhalten. Dies ist aus den Artikeln Angew. Chem. 106 (1994) 1146 von M.P. Kraff und J.G. Riess und Angew. Chem 100 (1988) 933 von H. Hoffmann und G. Ebert bekannt.

Die aus dem Stand der Technik bekannten fluorhaltigen Gele weisen darüber hinaus eine Affinität zu Wasser sowie zu Körpergewebe auf. Diese Wasser- und Gewebeaffinität führt bei langandauernder Verwendung in wässrigen Medien oder in Körpergewebe zur Auflösung und Zerstörung der Gele. Dies verhindert - in Verbindung mit der vorher beschriebenen Irreversibilität bei Zerstörung - die Langzeitanwendung als Implantat in Körpergewebe.

Es ist daher Aufgabe der vorliegenden Erfindung, ein plastisch verformbares Implantat zum Einbringen in natürliche oder künstliche Körperöffnungen des menschlichen oder tierischen Körpers bereitzustellen, welches auch eine Langzeitanwendung ermöglicht und Verwendungen eines solchen Implantats zur Herstellung von ophtalmologischen Hilfsmitteln und Arzneimitteln anzugeben.

Gelöst wird diese Aufgabe mit den kennzeichnenden Merkmalen des Anspruches 1. Vorteilhafte Ausgestaltungen und Verwendungen des erfindungsgemäßen Implantats sind den Unteransprüchen zu entnehmen.

Aufgrund ihrer vielseitigen und variablen Eigenschaften eignen sich die oben beschriebenen fluorhaltigen Gele als Grundstoff zur Ausbildung eines gattungsgemäßen Implantats. Für eine Langzeitanwendung muß jedoch zudem gewährleistet sein, daß sich das Implantat in wässrigen Medien nicht irreversibel auflöst. Weiterhin muß eine dauerhafte Stabilität gegen mechanische und thermische Beanspruchung vorhanden sein. Die Stabilität des Implantatstoffes bei Erhitzung muß insbesondere wegen der vor dem Einbringen in die Közperöffnungen notwendigen Sterilisation (121°C) gewährleistet sein. Eine der Erfindung zugrundeliegende Erkenntnis besteht darin, daß bei den fluorhaltigen Gelen gemäß des Anspruchs 1 die Gelstruktur nach einer überwiegenden Zerstörung des Gels vollständig reversibel rückbildbar ist. Dies stellt gemäß den bekannten Druckschriften über fluorhaltige Gele eine überraschende Erkenntnis dar.

In besonders vorteilhaften Ausführungsformen umfaßt das fluorhaltige Gel im wesentlichen drei Komponenten, nämlich ein Fluorcarbon, das fluorhaltige oberflächenaktive Tensid sowie Wasser. Den fluorcarbonhaltigen bzw. wässrigen Komponenten können hierbei verschiedene Zusätze beigefügt sein. Die erfindungsgemäße Kompositionszusammensetzungen von Tensiden, Fluorcarbonen und Wasser bilden Gele, welche ihre Gelstruktur nach einer erfolgten Verflüssigung, beispielsweise durch mechanischen

Druck oder Erhitzung, vollständig zurückbilden können. Diese Eigenschaft der erfindungsgemäßen Gele ermöglicht eine langandauernde Verwendung als gattungsgemäßes Implantat. Sollte es bei einem derartigen in eine Körperöffnung eingebrachten Implantat zu einer Verflüssigung des Implantat-Stoffes kommen, beispielsweise durch eine kurze Druckbelastung, so kann sich die Gelstruktur aufgrund der beschriebenen Reversibilität im Ruhezustand wieder herstellen. Das erfindungsgemäße Implantat besitzt somit einen selbstorganisierenden Heilungsmechanismus. Die Fähigkeit zu einem selbstorganisierenden Heilungsmechanismus kann beim Vorliegen eines Polyaphrongels auf die Stabilität der das Gel bildenden Aphrone zurückgeführt werden. Ein Gel kann sich nach Verflüssigung nur von selbst zurückbilden, wenn seine "Bausteine", die Aphrone, nicht vollständig zerstört wurden. Bleiben bei der Verflüssigung noch genügend intakte Aphrone bestehen, ist die Rückbildung möglich, wobei gerade die Übertragung der Aphronstruktur auf die homogenisierten Bereiche der umgebenden Flüssigkeit und damit ein Aufbau der Gelstruktur in der ganzen Flüssigkeit überraschend ist. Die Stabilität der Aphrone hängt von der Stärke der Wechselwirkung zwischen Wasser, Tensid und Perfluorcarbon ab, was wiederum durch Oberflächeneigenschaften und dem Vermögen zur Spreitung der einzelnen Phasen aufeinander determiniert ist. Ein wesentlicher Punkt ist zusätzlich die Stärke der Wechselwirkungen der Moleküle innerhalb der die Aphrone umhüllenden Filme (Wasser/Tensid; Perfluorcarbon/Tensid). Der selbstorganisierende Heilungsmechanismus stellt sich also nur dann ein, wenn die Oberflächeneigenschaften von Tensid/Wasser- bzw. Tensid/Perfluorcarbon-Film und der inneren Aphronphase aufeinander abgestimmt sind, d. h. die Tensidkraft die Aphronstruktur stabilisiert. Dies wird erreicht durch Verwendung von Fluortensiden der allgemeinen Formel

$$R_F\text{-}R_{pol},$$

wobei $R_F$ lineare oder verzweigte Perfluoralkylgruppen mit mehr als 5 Kohlenstoffatomen und $R_{pol}$ ein polarer Kohlenwasserstoffrest ist, der mindestens eine funktionelle Gruppe enthält, die ausgewählt ist aus $CO\text{-}NH(R)$, $CO\text{-}N(R)_2$, $COO\text{-}$, $COOR$, $SO_3\text{-}$; $SO_2N(R)_2$, $CH_2\text{-}O\text{-}R$, $PO_2H$, $PO_3H$. Die Molmasse ist vorzugsweise > 400 g/mol, die Oberflächenspannung in wässriger Lösung < 30mN/m und vorzugsweise < 20mN/m. Die Grenzflächenspannung in wässriger Lösung zur unpolaren Komponente ist < 25 mN/m, vorzugsweise < 10 mN/m und die Konzentration < 0,3%, vorzugsweise < 0,1%. Dies kann bei nicht fluorierten Tensiden durch einen starken cohesiven Effekt erreicht werden mit einem HLB Wert von über 25 (HLB=hydrophilic-lipophilic-balance nach Griffin in J.Soc.Cosmet.Chem 1 (1949) 311).

Das erfindungsgemäße Implantat kann damit sowohl thermischer Belastung, beispielsweise bei der Sterilisation, als auch mechanischer Belastung, beispielsweise durch Druck auf die Körperöffnung, widerstehen. Die strukturelle Reversibilität der erfindungsgemäßen Implantate verhindert darüber hinaus die Zerstörung des Implantatwerkstoffes durch Diffusionsprozesse in den Körperhöhlen. Die üblicherweise durch diese Diffusionsprozesse stark beeinträchtigte Transparenz der fluorhaltigen Gele bleibt bei den erfindungsgemäßen Implantaten im dynamischen Gleichgewicht vollständig erhalten.

Die Biokompatibilität der erfindungsgemäßen Implantate ist durch Verwendung von hochgereinigten Ausgangsstoffen und durch die Verwendung sehr geringer Tensidmengen (vorzugsweise < 0,1%) gegeben. Außerdem sind die verwendeten Tenside gut gewebeverträglich, fest im Gel gebunden und über das gesamte Volumen des Implantats homogen verteilt.

Das erfindungsgemäße Implantat kommt beispielsweise in der Ophthalmologie als Glaskörperersatz zur Anwendung. Hierfür sind insbesondere fluorhaltige Gele mit hohem spezifischen Gewicht und gleichzeitig hoher Affinität für wasserlösliche Stoffe gut geeignet. Damit wird erstmalig ein Tamponademittel bzw. Implantat mit höheren spezifischem Gewicht als Wasser bei gleichzeitigem Vermögen zur Aufnahme von wasscrlöslichen Ionen bereitgestellt. Nach erfolgter Vitrektomie und üblicher Verfahren der Netzhautchirurgie wird das formveränderliche Implantat in den Glaskörperraum injiziert. Durch Aufnahme von Wasser expandiert das plastisch verformbare Implantat.

Die durch die Wasseraufnahme bedingte Volumenvergrößerung unterstützt dabei den durch die hochdichten Fluorcarbone bewirkten Tamponadeeffekt. Gleichzeitig baut sich im Innern des Implantats ein Druck auf, der einer weiteren Volumenexpansion und Wasseraufnahme entgegenwirkt. Das sich dadurch einstellende dynamische Gleichgewicht wird durch die strukturelle Reversibiltät des Implantatstoffes gesichert und ermöglicht damit eine Langzeitanwendung des Implantats.

Ein weiterer Vorteil des erfindungsgemäßen Implantats in der Verwendung als Glaskörperersatz ist die Reduzierung von mechanischen Schädigungen im Bereich der Retina. Solche Schädigungen sind bei der Verwendung von reinen Fluorcarbonen als Glaskörperersatzstoffe bekannt und wurden allein auf die hohe Dichte der Fluorcarbone zurückgeführt. Wie sich erst jetzt zeigt, ist für die Schädigung nicht der statische Druck verantwortlich. Vielmehr kommen Schädigungen dadurch zustande, weil das Auftreffen von schweren Fluiden auf die Retina, beispielsweise bei schnellen Kopfbewegungen, mechanische Druckstöße verursacht. Bei der Verwendung von fluorhaltigen Gelen als Glaskörperersatzstoff kann durch die Verwendung bestimmter Gele dieser Effekt verhindert werden. Bei diesen Gelen handelt es sich um Gele mit einem hohem Viskositäts-/Dichtequotienten von > 100 mPas cm$^3$/g, vorzugsweise > 1000 mPas cm$^3$/g. Derartige erfindungsgemäße Gele ermöglichen die Tamponade im unteren Augensegment, ohne daß bewegungsbedingte Druckspitzen bei ruckartigen Bewegungen auftreten. Dies wird durch die - verglichen mit reinen Fluorcarbonen - erhöhte Viskosität bewirkt, welche den Beschleunigungskräften entgegenwirkt und ein schädigendes Auftreffen schwerer Fluide auf die Retina verhindert. Hierbei erweist es sich als besonders vorteilhaft, daß bei den fluorhaltigen Gelen die Stoffeigenschaften über die Komposition in weiten Grenzen variabel ist, verglichen mit den reinen Fluorcarbonen.

Durch Verwendung der erfindungsgemäßen Implantate als ophthalmologische Behandlungsmittel für den vitreo retinalen Bereich, kann im Gegensatz zu allen bisher beschriebenen ophthalmologischen Behandlungsmitteln auf Basis fluorierter Verbindungen nicht nur vorrangig eine Wiederanlegung der Netzhaut und eine Kurzzeittamponade erreicht werden. Vielmehr können neben der tamponierenden Wirkung noch weitere Funktionen des natürlichen Glaskörpers übernommen werden. Damit ergibt sich auch die Möglichkeit einer Behandlung krankhafter Veränderungen im vitreo retinalen Bereich bzw. die Möglichkeit der Unterdrückung von Prozessen, die zu einer nachhaltigen Schädigung der Netzhaut führen können, z.B. zu Müllerzellenschädigungen. Dafür können die Behandlungsmittel so ausgestaltet werden, daß sie verschiedene, auch gegenläufige Eigenschaften in der Weise in sich vereinigen, daß diese in einen Behandlungsschritt zur Wirkung gebracht werden. Die Verwendungsmöglichkeiten der Gele werden durch die in den Gelen enthaltenen Fluorcarbone unterstützt und erweitert, die bekanntermaßen besondere Eigenschaften wie antinflammatorische und gaslösende Eigenschaften besitzen.

Die weiteren von fluorhaltigen Verbindungen bekannten Eigenschaften, welche in der Anwendung als ophthalmologisches Hilfsmittel von Vorteil sind, bleiben bei den erfindungsgemäßen Implantaten erhalten oder werden sogar noch verstärkt. So z. B. die Möglichkeit der Laserbehandlung, die Tamponadeeigenschaften, sowie die Löslichkeiten von Wirkstoffen. Die Entfernung der erfindungsgemäßen Implantate aus den Körperöffnungen kann nach üblichen Verfahren, beispielsweise entsprechend einer Vitrektomie, durchgeführt werden.

Die erfindungsgemäßen fluorhaltigen Implantate eignen sich auch in der Verwendung als Intraokularlinsen. Für diesen Einsatzzweck sind hochtransparente Gele zu verwenden, die einen besonders hohen Viskositäts-/Dichtequotienten besitzen, was insbesondere durch den Einsatz von oligomeren, $R_F R_H$-Verbindungen als diskontinuierliche Phase, beispielsweise beschrieben in der EP-A 545 174, erreicht wird. Außerdem ist die Brechzahl der verwendeten Gele im Bereich von 1,33 - 1,34, insbesondere im Bereich von 1,334 - 1,338 einzustellen, was beispielsweise bei Verwendung der folgenden Verbindungen gelingt:

| Fluorcarbon | Tensid Name/Struktur //Abkürzung// Charakteristik | Brechungs-index | Bioverträglichkeit (Draizetest) |
|---|---|---|---|
| Perfluorphenanthren | Perfluoralkylethanol-Oxethylat (Fluowet OTN, Clariant) $\sigma_O$=18mNm $\sigma_G$=19mNm | 1,3357 | n.b. |
| Perfluorphenanthren | Fluoriertes Aminoxid (Fluowet OX, Clariant) $\sigma_O$=22mNm $\sigma_G$=12mNm | 1,3361 | n.b. |
| Perfluorphenanthren | Perfluoralkylethanol-Oxethylat (Fluowet OTL, Clariant) $\sigma_o$=19mNm; $\sigma_G$=10mNm | 1,3355 | o.B. |
| Perfluorphenanthren | Perfluoroctansäure-tetraethylpiperaziniumsalz (HO224 $\sigma_o$=16mNm | 1,3362 | o.B. |
| Perfluorphenanthren | Perfluoroctansäure-N-methyl-D-glucamid (T14) $\sigma_o$ <20mNm | 1,3360 | o.B. |
| Perfluorphenanthren | Perlluoooctansäure-diethanolamid (HO31) $\sigma_o$ <20mNm | 1,3358 | o.B. |
| Perfluorphenanthren | Teramethylammoniumsalz der Perfluoroctansäure (E749) $\sigma_o$ <20mNm | 1,336 | o.B. |

(fortgesetzt)

| Fluorcarbon | Tensid Name/Struktur //Abkürzung// Charakteristik | Brechungs-index | Bioverträglichkeit (Draizetest) |
|---|---|---|---|
| Perfluorphenanthren | Perfluoroctansäureamido-trimethylammoniumiodid (B98) $\sigma_O$ <20mNm | 1,336 | o.B. |
| Perfluorphenanthren | Tetraethylammoniumsalz der Perfluoroctansulfonsäure(B248) $\sigma_O$ <20mNm | 1,3359 | o.B. |
| Perfluorphenanthren | Perfluordecansäure-N-(2-hydroxyethyl)-D-glucamid (T21) $\sigma_O$ <20mNm | 1,3357 | o.B. |
| Perfluorphenanthren | Perfluoroctansäure-N-(2-hydroxyethyl)-D-glucamid (T16) $\sigma_O$ <20mNm | 1,336 | o.B. |
| $C_6F_{13}C_8H_{17}$ | Tetramethylammoniumsalz der Perfluoroctansäure (E749) $\sigma_O$ <20mNm | 1,3463 | n.b. |
| $(C_6F_{13}C_2H_4)_3$ | Tetramethylammoniumsalz der Perfluoroctansäure (E749) $\sigma_O$ <20mNm | 1,3357 | n.b. |

o.B. = ohne Befund

n.b. = nicht bestimmt

$\sigma_O$ = Oberflächenspannung

$\sigma_G$ = Grenflächenspannung
     zur unpol. Komp.

Die erfindungsgemäßen Implantate können anstelle der üblicherweise bei Staroperationen verwendeten künstlichen Intraokularlinsen aus Silikon, PMMA oder Akryl eingesetzt werden. Hierbei wird nach Eröffnung des Kapselsackes und Entfernung der getrübten, natürlichen Linse nach bekannten Verfahren der Implantatwerkstoff eingespritzt, so daß dieser den gesamten Kapselsack vollständig ausfüllt. Das Implantat übernimmt damit die komplette Funktion der natürlichen Linse, d. h. trotz Staroperation bleibt die Akkommodationsfähigkeit der Linse erhalten. Wegen der ständig auf das Implantat einwirkenden Kräfte ist die mechanische Langzeitstabilität bei dieser Anwendung von ganz besonderer Bedeutung.

Weitere Anwendungen der erfindungsgemäßen Implantate finden sich auf dem Gebiet der temporären Versiegelung von Körperhöhlen und der temporären Trennung von Gewebepartien, beispielsweise bei der Nutzung der Implantate als Expander oder zur Anregung von Knochenwachstum. In der Zahnmedizin kommt das erfindungsgemäße Implantat insbesondere zur temporären Füllung von Extraktionshöhlen im Kieferknochen und als Gewebeexpander zur Verwendung. Weiterhin kommt eine orthopädische Verwendung als biokompatibler Gleitfilm für Gelenke und Gelenkprothesen in Betracht. Nach der Einbringung des Implantat-Werkstoffes in die Extraktionshöhlen werden diese durch Zusammennähen des umgebenden Gewebes gekapselt. Somit wird ein Herauslaufen des gelförmigen Implantats verhindert.

Die im folgenden beschriebenen Ausführungsbeispiele sollen die Auswahl der Implantatwerkstoffe sowie deren Herstellung näher erläutern. Hierbei wird auf die begleitenden Zeichnungen Bezug genommen. Die Zeichnungen zeigen:

**Fig. 1:** Messung von Druckspitzen beim Beschleunigen von Perfluorphenanthren in einem verschlossenen Glasrohr, Vollausschlag entspricht 70 mbar (52,5 mm Hg).

**Fig. 2:** Messung von Druckspitzen beim Beschleunigen eines erfindungsgemäßen Implantatwerkstoffes in einem verschlossenen Glasrohr, Vollausschlag entspricht 70 mbar (52,5 mm Hg).

Beispiel 1

Aus nach bekannten Verfahren (EP 0 626 936 B1) hochgereinigtem Perfluorphenanthren, isotonischer Kochsalzlösung und Fluowet OTL (Firma Clariant) wird durch Ultraschall eine Mischung aus 99% Fluorcarbon, 0,9% isotonischer Kochsalzlösung und 0,1% OTL hergestellt; zu dieser Lösung wird ein nach üblichen Verfahren hergestelltes Polyaphrongel in einer Volumenkonzentration von unter 30 % langsam eingeführt, bis die gesamte Mischung zu einem Gel erstarrt. Durch vorsichtiges Entgasen oder Zentrifugieren wird die Präparation vollständig transparent.

Durch mechanische Wechselbelastung, Erhitzen auf ca. 130° C oder Zusatz von Wasser, kann dieser Implantatwerkstoff vollständig verflüssigt werden. Durch Zufuhr leichter mechanischer Energie bzw. Abkühlen bzw. Wasserentzug durch ein saugfähiges Material (trockener Glasfilter etc.) wird das Gel in seinen ursprünglichen Zustand gebracht. Diese Prozedur kann mehrmals wiederholt werden, ohne daß sich die Zusammensetzung des formveränderlichen Implantatwerkstoffes ändert.

Beispiel 2

Ein nach Beispiel 1 hergestellter formveränderlicher Implantatwerkstoff wird mit der doppelten Menge Wasser überschichtet. Dadurch dehnt sich die gelförmige Phase aus. Wird das Volumen z. B. durch semipermeable Böden begrenzt, baut sich im Inneren des Werkstoffes ein erhöhter Druck so lange auf, bis das von einer Seite beaufschlagte Wasser auf der anderen Seite austritt und abfließt, ohne daß die Gelstruktur zerstört wird.

Beispiel 3

Ein nach Beispiel 1 hergestellter Implantatwerkstoff, der an Stelle von OTL (Fa. Clariant) T14 enthält, wird mit der dreifachen Menge an Wasser überschichtet. Anstelle der ursprünglichen Phasengrenze bildet sich eine dünne dritte Phase aus. Durch besondere Abstimmung der Diffusionsgeschwindigkeiten aus der Grenzschicht des Implantats und der Tiefe des Volumens des Gels bildet sich eine Perfluorphenanthren-Sperrschicht, die eine weitere Verdünnung oder den Abbau des Gels verhindert. Dadurch wird eine extreme Langzeitstabilität erreicht.

Beispiel 4

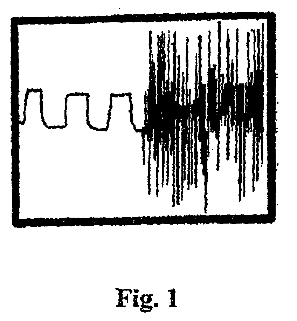

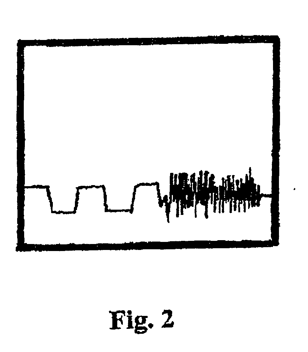

Ein nach Beispiel 1 hergestelltes Gel wird in ein zweiseitig verschlossenes Glasrohr gegeben. An einer Seite des Glasrohres wird ein empfindlicher Drucksensor angekoppelt. Das Glasrohr wird danach geschüttelt oder so positioniert, daß wechselseitig eine Öffnung nach unten zeigt. Der gleiche Versuch wird wiederholt, indem anstelle des formveränderlichen Implantats Wasser bzw. Perfluorphenanthren eingesetzt wird (Fig.1). Durch den Viskositäts-/Dichtequotienten von >3000 mPas cm$^3$/g kann durch den Implantatwerkstoff eine den Perfluorcarbonen entsprechende Tamponadewirkung erzeugen, ohne daß die bei Schleuder- oder Schüttelbewegungen auftretenden Druckspitzen (bei unvollständiger Füllung bis zu 50 mm Quecksilbersäule) wie sie bei reinen Perfluorcarbonen auftreten, gemessen werden können. Bei ophthalmologischen Anwendungen dürfen die Druckspitzen den tolerierbaren Augeninnendruck nicht übersteigen (20 - kurzzeitig 30 mm Quecksilbersäule). Damit weist das formveränderliche Implantat eine für den ophthalmologischen Einsatz günstige Charakterisitik auf, die für den Langzeiteinsatz als Glaskörperersatzstoff notwendig ist und mechanische Schädigungen an der Retina verhindern können (Fig. 2).

Beispiel 5

Nach dem in Beispiel 1 beschriebenen Verfahren können als Tenside zur Herstellung erfindungsgemäßer Implantate z. B. Natriumdodecylsulfat (SDS) HLB 40 oder Pluoronic F68 (F68) HLB 29 verwendet werden. In beiden Fällen wird als Fluorcarbon Perfluorophenanthren verwendet. Die Substanzen sind bei 121°C sterilisierbar.

**Patentansprüche**

**1.** Plastisch verformbares Implantat zum Einbringen in Körperöffnungen des menschlichen oder tierischen Körpers, welches von einem nicht umschlossenen, unmittelbar in die natürliche oder künstlich gelegte Körperöffnung einzubringendes Gel gebildet ist, welches ein Fluorcarbon enthält, wobei das Gel Polyaphronstruktur besitzt und neben dem Fluorcarbon Wasser und mindestens ein Tensid enthält, welches ein Fluortensid der allgemeinen Formel $R_F$-$R_{pol}$ ist, wobei $R_F$ lineare oder verzweigte Perfluoralkylgruppen mit mehr als 5 Kohlenstoffatomen sind und $R_{pol}$ ein polarer Kohlenwasserstoffrest mit mindestens einer funktionellen Gruppe, ausgewählt aus CO-NH(R), CO-N(R)$_2$, COO-, COOR, SO$_3$-; SO$_2$N(R)$_2$, CH$_2$-O-R, PO$_2$H, PO$_3$H (R=alkyl) mit einer Molmasse > 400 g/mol, einer Oberflächenspannung der wässrigen Lösung < 30 mN/m, einer Grenzflächenspannung in wässriger Lösung zur unpolaren Komponente < 25 mN/m und einer Konzentration < 0,3%.

2. Implantat nach Anspruch 1, **dadurch gekennzeichnet, daß** das Fluorcarbon ein Perfluorcarbon und/oder ein teilfluoriertes Alkan ist.

3. Implantat nach einem der voranstehenden Ansprüche, **dadurch gekennzeichnet, daß** das Fluorcarbon ein Oligomer ist.

4. Implantat nach einem der vorstehenden Ansprüche, **dadurch gekennzeichnet, daß** das Tensid fluorcarbonlöslich ist, lineare oder verzweigte Perfluoralkylgruppen mit mehr als, 5 Kohlenstoffatomen enthält und die Fluorcarbon/ Tensid-Komponente weniger als 30% Fluortensid enthält.

5. Implantat nach einem der voranstehenden Ansprüche, **dadurch gekennzeichnet, daß** der Quotient aus Viskosität und Dichte des Gels größer ist als 0,1 Pas cm$^3$/g und kleiner als 3 Pas cm$^3$/g, vorzugsweise kleiner als 1 Pas cm$^3$/g.

6. Implantat nach einem der voranstehenden Ansprüche, **dadurch gekennzeichnet, daß** die Struktur des Gels nach einer Verflüssigung vollständig reversibel rückbildbar ist.

7. Verwendung eines Implantats nach einem der voranstehenden Ansprüche zur Herstellung eines ophtalmologischen Hilfsmittels, insbesondere eines Glaskörpers - oder Linsenersatzes.

8. Verwendung nach Anspruch 7, **dadurch gekennzeichnet, daß** der Brechungsindex zwischen 1,334 und 1,338 liegt, das spezifische Gewicht größer als 1,05 g/cm$^3$ ist sowie Permeabilität für wasserlösliche und ionische Verbindungen besteht.

9. Verwendung eines Implantats nach einem der Ansprüche 1 bis 6 zur Herstellung eines zahnmedizinischen Hilfsmittels, insbesondere zum Zwecke der Füllung von Extraktionshöhlen im Kieferknochen.

10. Verwendung eines Implantats nach einem der Ansprüche 1 bis 6 zur Herstellung eines Arzneimittels zur Sauerstofftherapie des die Körperöffnung umgebenden Gewebes.

11. Verwendung eines Implantats nach einem der Ansprüche 1 bis 6 zur Herstellung eines gewebeexpandierenden Arzneimittels.

**Claims**

1. Plastically deformable implant for insertion into bodily orifices of the human or animal body which is formed from a gel which is not surrounded and can be introduced directly into the natural or artificially made orifice, and which comprises a fluorocarbon, wherein the gel has a polyaphron structure and includes water and at least one surfactant besides the fluorocarbon, wherein the surfactant is a fluorinated surfactant of the general formula $R_F$-$R_{pol}$, wherein $R_F$ represents linear or branched perfluoroalkyl groups having more than 5 carbon atoms and $R_{pol}$ represents a polar hydrocarbon residue having at least one functional group selected from CO-NH(R), CO-N(R)$_2$, COO-, COOR, SO$_3$-; SO$_2$N(R)$_2$, CH$_2$-O-R, PO$_2$H, PO$_3$H (R=alkyl) having a molar mass of > 400 g/mol, a surface tension of the aqueous solution of < 30 mN/m, an interfacial tension in the aqueous solution to the nonpolar component of < 25 mN/m and a concentration of < 0.3%.

2. Implant according to claim 1,
**characterized in that** the fluorocarbon is a perfluorocarbon and/or a partially fluorinated alkane.

3. Implant according to any one of the previous claims,
**characterized in that** the fluorocarbon is an oligomer.

4. Implant according to any one of the previous claims,
**characterized in that** the surfactant is soluble in fluorocarbons, and contains linear or branched perfluoroalkyl groups having more than 5 carbon atoms, and the fluorocarbon/surfactant component includes less than 30% of fluorinated surfactant.

**5.** Implant according to any one of the previous claims,
**characterized in that** the quotient of viscosity and density of the gel is more than 0.1 Pas cm$^3$/g and less than 3 Pas cm$^3$/g, preferably less than 1 Pas cm$^3$/g.

**6.** Implant according to any one of the previous claims,
**characterized in that** the structure of the gel is completely reversibly re-formable after liquefaction.

**7.** Use of an implant according to any one of the previous claims for the manufacture of an ophthalmologic adjuvant, in particular of a vitreous body or lens substitute.

**8.** Use according to claim 7, **characterized in that** the refractive index is between 1.334 and 1.338, the specific weight is more than 1.05 g/cm$^3$ and permeability for water soluble and ionic compounds exists.

**9.** Use of an implant according to any one of claims 1 to 6 for the manufacture of a dental aid, in particular for the purpose of filling of extraction fissures in the jaw bone.

**10.** Use of an implant according to any one of claims 1 to 6 for the manufacture of a medicament for oxygen therapy of the tissue surrounding the orifice.

**11.** Use of an implant according to any one of claims 1 to 6 for the manufacture of a tissue-expanding medicament.

**Revendications**

**1.** Implant déformable plastiquement, destiné à être introduit dans des orifices corporels de l'organisme humain ou animal, qui est formé par un gel non enfermé à introduire directement dans l'orifice corporel naturel ou réalisé artificiellement, lequel gel contient un fluorocarbone, possède une structure polyaphroïque et contient, en plus du fluorocarbone, de l'eau et au moins un agent tensioactif qui est un agent tensioactif fluoré de formule générale $R_F$-$R_{pol}$, dans laquelle $R_F$ représente des groupes perfluoroalkyle linéaires ou ramifiés ayant plus de 5 atomes de carbone et $R_{pol}$ représente un résidu hydrocarbure polaire possédant au moins un groupe fonctionnel choisi parmi CO-NH(R), CO-N(R)$_2$, COO-, COOR, SO$_3$-, SO$_2$N(R)$_2$, CH$_2$-O-R, PO$_2$H, PO$_3$H (R = alkyle) avec une masse molaire supérieure à 400 g/mol, une tension de surface de la solution aqueuse inférieure à 30 mN/m, une tension interfaciale en solution aqueuse par rapport au composant non polaire inférieure à 25 mN/m et une concentration inférieure à 0,3 %.

**2.** Implant selon la revendication 1, **caractérisé en ce que** le fluorocarbone est un perfluorocarbone et/ou un alcane partiellement fluoré.

**3.** Implant selon l'une des revendications précédentes, **caractérisé en ce que** le fluorocarbone est un oligomère.

**4.** Implant selon l'une des revendications précédentes, **caractérisé en ce que** l'agent tensioactif est soluble dans le fluorocarbone et contient des groupes perfluoroalkyle linéaires ou ramifiés ayant plus de 5 atomes de carbone et **en ce que** le composant fluorocarbone/agent tensioactif contient moins de 30 % d'agent tensioactif fluoré.

**5.** Implant selon l'une des revendications précédentes, **caractérisé en ce que** le quotient de la viscosité et de la densité du gel est supérieur à 0,1 Pa s cm$^3$/g et inférieur à 3 Pa s cm$^3$/g, de préférence inférieur à 1 Pa s cm$^3$/g.

**6.** Implant selon l'une des revendications précédentes, **caractérisé en ce que** la structure du gel peut être reformée de manière entièrement réversible après une liquéfaction.

**7.** Utilisation d'un implant selon l'une des revendications précédentes pour préparer un auxiliaire ophtalmologique, en particulier un substitut du vitré ou du cristallin.

**8.** Utilisation selon la revendication 7, **caractérisée en ce que** l'indice de réfraction se situe dans la plage de 1,334 à 1,338 et **en ce que** la masse volumique est supérieure à 1,05 g/cm$^3$ et possède une perméabilité pour des composés solubles dans l'eau et ioniques.

**9.** Utilisation d'un implant selon l'une des revendications 1 à 6 pour préparer un auxiliaire médico-dentaire, en particulier à des fins de remplissage de cavités d'extraction dans l'os de la mâchoire.

10. Utilisation d'un implant selon l'une des revendications 1 à 6 pour préparer un médicament destiné à l'oxygénothérapie des tissus qui entourent l'orifice corporel.

11. Utilisation d'un implant selon l'une des revendications 1 à 6 pour préparer un médicament dilatant les tissus.

Fig. 1

Fig. 2